# EUROPEAN PATENT SPECIFICATION

(11) **EP 0 606 318 B1**
(45) Date of publication and mention of the grant of the patent: **28.01.1998**
(21) Application number: 92920526.8
(22) Date of filing: 29.09.1992
(51) Int. Cl.: A61K 31/555

(54) **VANADIUM COMPOSITIONS**
VANADIUM ENTHALTENDE ZUSAMMENSETZUNGEN
COMPOSITIONS CONTENANT DU VANADIUM

(30) Priority: 30.09.1991 US 767510
(43) Date of publication of application: 20.07.1994
(60) Divisional application: 97200558.1
(73) Proprietor: THE UNIVERSITY OF BRITISH COLUMBIA, Vancouver, British Columbia V6T 1Z1 (CA)
(72) Inventor: McNEILL, John Hugh, Delta, British Columbia V4M 2K4 (CA); HOVEYDA, Hamid Reza, Vancouver, British Columbia, V5Y 3B6 (CA); ORVIG, Chris, Vancouver, British Columbia V6M 2G2 (CA)
(74) Representative: Cockbain, Julian, Dr.
(86) International application number: PCT/EP92/02235
(87) International publication number: WO 93/06811

(56) References cited:
- Week 0103, Derwent Publications Ltd., London, GB; AN 91-019158; & JP-A-2 292 217
- J. CLIN. BIOCHEM. NUTR., vol. 8, no. 3, 1990, pages 193-200; H. SAKURAI ET AL.: 'Iinsulin-mimetic action of vanadyl complexes.'
- J. MED. CHEM., vol. 35, no. 8, 1992, pages 1489-1491; J.H. MCNEILL ET AL.: 'Bis (maltolato)oxovanadium(IV) is a potent insulin mimic.'
- ZEITSCHR. ANORG. ALLG. CHEM., vol. 373, no. 2, 1970, pages 204-208; A. SYAMAL: "Some oxovanadium(IV) complexes with C2v symmetry"
- J. INDIAN. CHEM. SOC., vol. 44, no. 5, 1967, pages 381-387; R.L. DUTTA ET AL.: 'Vanadium. part XV. Oxovanadium(IV) and oxovanadium(V) complexes of some organic ligands'

## Description

This invention relates to pharmaceutical compositions useful for lowering blood sugar in mammals.

Diabetes mellitus is a mammalian condition in which the amount of glucose in the blood plasma is abnormally high. This condition can be life-threatening and high glucose levels in the blood plasma (hyperglycemia) can lead to a number of chronic diabetes syndromes, for example, atherosclerosis, microangiopathy, kidney disorders, renal failure, cardiac disease, diabetic retinopathy and other ocular disorders including blindness.

It is a disease of some complexity, as indicated by its effect on a large number of important functions of the body. There are large numbers of sufferers. For example, in the late 1980's over 2.6 million people in the United States were diabetics who were taking insulin by injection daily. Approximately an equal number of diabetics were taking oral hypoglycemic agents and another 2 to 3 million people were controlling the disease by dietary methods alone. It is estimated there are several million people in the U.S. who are undiagnosed diabetics.

In non-diabetics, plasma glucose level is maintained automatically in a complex procedure that involves, inter alia, the hormone insulin. In diabetes, external intervention is needed. Treatment of diabetics is now carried out using several drugs. Insulin is the mainstay of treatment; it replaces the natural hormone produced in the pancreas. In diabetics, insulin is not produced in sufficient quantities, or the body becomes tolerant to insulin and requires more than normal amounts to produce the necessary effect.

Insulin must be given by injection. Insulin cannot be administered orally as it is decomposed before or during passage through the gastrointestinal tract. It is difficult to determine the exact amount required. This can result in overdoses, leading to hypoglycemia, and in inadequate doses, leading to poor control of the disease and the development of secondary complications.

Oral diabetes medications (such as sulphonylureas and biguanides) are available. As with insulin it can be difficult to obtain a correct dose. There has been shown to be a difficulty with the sulphonylureas in correctly regulating blood glucose levels. Hypoglycemic episodes often occur in elderly patients. The biguanides lower blood glucose, but can produce side effects, such as lactic acidosis, which can be fatal.

Sodium orthovanadate has been found to be a potent inhibitor of Na⁺-K⁺ATPase. It has also been found that vanadate (vanadium (V)) taken up by the red blood cells was reduced to vanadium (IV) in the form of vanadyl ion VO²⁺ in the cytoplasm. Since this work, there has been interest in the effects of vanadium, mostly as vanadate, on glucose metabolism and uptake into cells. A natural outgrowth of this work has been the study of vanadium and diabetes. (See J. Biol. Chem. 252: 7421-7423 (1977), J. Biol. Chem. 254: 1781-1784 (1979) and Diabetes 39: 1-5 (1990)).

The insulin-like effect of the vanadate ion (VO₄³⁻) in vitro has been known since 1980 (see Nature 284: 556-558 (1980)) when it was shown that the insulin-like stimulation of glucose oxidation in rat adipocytes was due to the vanadyl ion. In 1985, McNeill et al. (Science 227: 1474-1477 (1985)) reported that vanadate, when administered in drinking water, decreased the elevated blood glucose and prevented the depression of cardiac performance in rats made diabetic with streptozotocin (STZ). Subsequently, there has been interest in the insulin-mimetic effects of both vanadate and vanadyl since Sakurai et al. showed that vanadate is reduced in vivo to vanadyl (Biochem. Biophys. Res. Comm. 96: 293-298 (1980)).

Vanadate however has the drawbacks that it is poorly absorbed from the gastrointestinal tract to the blood and that it is toxic. Administered concentrations must be close to the toxic level if the insulin-mimetic effects in mammals are to be achieved.

Work by Sakurai et al. (see J. Clin. Biochem. Nutr. 8: 193-200 (1990)) showed that the blood glucose levels of STZ-induced diabetic rats dropped from hyperglycemic levels to hypoglycemic levels within 24-48 h after treatment with vanadyl sulfate by intraperitoneal injection.

Work by McNeill et al. (see Am. J. Physiol 257: H904-H911 (1989), Metabolism 38: 1022-1028 (1989), Diabetes 38: 1390-1395 (1989) and Can. J. Physiol & Pharmacol. 68: 486-491 (1990)) has shown that vanadyl administered orally as vanadyl sulfate also lowers blood glucose and blood lipids in STZ diabetic rats and prevents secondary complications of diabetes such as cataracts and cardiac dysfunction. Vanadyl sulfate is less toxic than the vanadate form of vanadium but is also poorly absorbed. There have been attempts to modify the biological uptake of vanadium by changing the chemical form in which it is supplied from either vanadate (VO₄³⁻) or vanadyl sulfate (VOSO₄.(H₂O)ₓ). In this regard, the use of various vanadate:peroxide reaction products as insulin mimics has been suggested by Posner et al. (See US-A-4882171, US-A-5069913 Biochem. Biophys. Res. Comm. 147: 259-266 (1987) and J. Biol. Chem. 262: 8252-8256 (1987)).

The use, for the oral treatment of diabetes, of a cysteine complex of vanadium of the formula 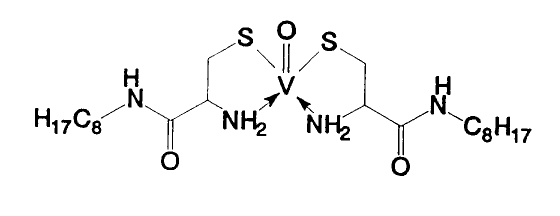 is described in EP-A-305264. The use of vanadium cysteine complexes was also reported in JP-A-2/292217 by Komatsu et al. However these cysteine complexes are only poorly water soluble and thus are not suited to oral administration. Moreover their utility is further diminished by the relatively long delay between administration and the onset of a blood sugar lowering effect. Komatsu et al. (supra) also proposed the use of a pentane-2,4-dione complex of the vanadyl ion. This however is hydrophobic and insoluble in water and thus too is unsuited to oral administration.

Accordingly, there remains a need for compositions useful in the treatment of diabetes with vanadium and we have found that certain bidentate, monoprotic chelants, with vanadium co-ordinating oxygen, sulphur or nitrogen atoms form vanadium complexes particularly suitable for therapeutic use, especially for oral administration.

We have found that certain vanadium compounds are particularly effective in this regard.

Thus viewed from one aspect the invention provides a pharmaceutical composition, preferably in a form adapted for administration into the gastrointestinal tract, comprising at least one physiologically acceptable carrier together with a physiologically tolerable vanadium complex of a bidentate monoprotic α-hydroxypyrone or α-hydroxypyridinone chelant capable of chelating vanadium to form a five-membered, unsaturated, vanadium-containing ring, said ring containing at least two further heteroatoms besides vanadium.

Viewed from a further aspect the invention provides the use of a vanadium complex as defined above for the manufacture of a therapeutic agent for lowering blood sugar levels.

The five-membered rings formed by chelation of vanadium by the bidentate monoprotic chelant used according to the invention preferably involves oxygen or oxygen and nitrogen coordination of the vanadium, and preferably oxygen coordination alone.

Particularly suitable chelants are the α-hydroxy-pyridinones of formula 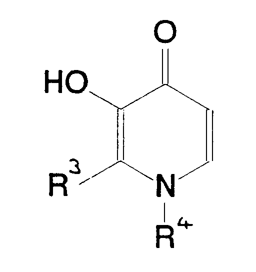 and the α-hydroxypyrones of formula 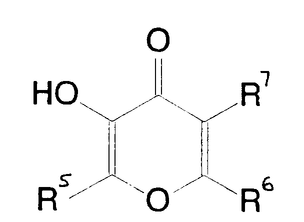 wherein R³ to R⁶ are hydrogen or optionally substituted, e.g. hydroxylated, C₁₋₄ alkyl.

The especially preferred chelant is maltol.

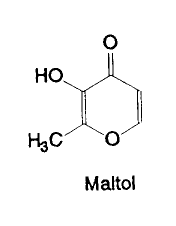

Chelants of this type are known from the literature (see for example Matsuba et al Inorg. Chem. 27: 3935-3939 (1988)) or may be produced from known starting materials by conventional techniques.

Many of the vanadium chelates with ligands of formula I might be prepared from maltol or maltol derivatives in a one-pot synthesis analogous to that described for gallium, aluminium and indium complexes by Zhang et al in Can. J. Chem. 67: 1708-1710 (1989). Thus

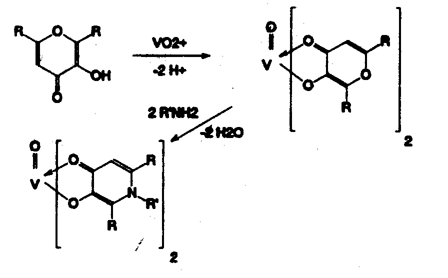

The direct electrochemical preparation of a vanadyl maltol compound was reported in J. Coord. Chem. 8: 27-33 (1978). The biological activity was not examined and the structural formula was not determined but the data suggested it to be:

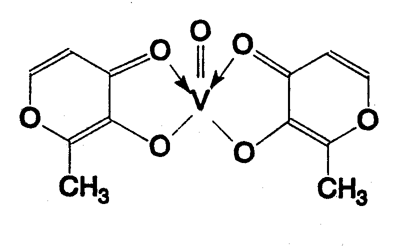

Alkoxo-oxovanadium (V) derivatives of the maltolate anion have been known since the 1960's, but again the biological activity was not examined (see Z. Anal. Chem. 203: 257-260 (1964)).

The vanadium complex used according to the invention is preferably a complex of vanadium (IV) or (V) with a ligand as described above, especially preferably a complex VOL₂ or VOL₂(OR²⁰) where LH is a chelate as described above and R²⁰ is an optionally substituted alkyl, aryl, aralkyl or alkenyl group (where such groups are as defined above), preferably such a group is substituted by at least one hydroxyl group.

Where LH is a chelant which forms a five membered C=O→V-O-C ring, these VOL₂ and VOL₂(OR²⁰) complexes, can schematically be represented by the formulae 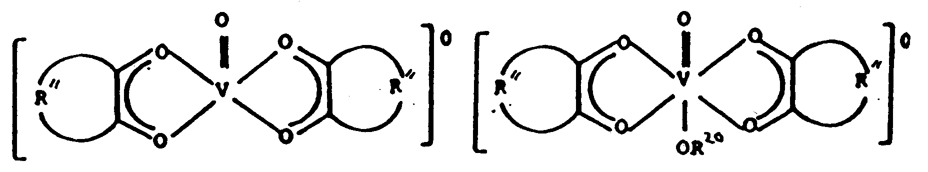 where R'' is the remainder of the chelating group.

In general, backbone and ring substituents on the vanadium coordinating ligands are selected for their solubilizing effect. Desirably the vanadium complexes used according to the invention will be neutrally charged overall, water soluble (eg. capable of forming an at least 0.1 mM solution, preferably an at least 0.2 mM solution), orally absorbable (preferably with significant gastro-intestinal absorption), and have moderate to high complex stability (eg. with ligand binding constants log β₂ ≥ 2 to 30, preferably 6 to 20).

A particularly preferred compound is the maltol complex bis(maltolato)oxovanadium (IV).

The vanadium compounds can be given by conventional administration routes, eg. oral, rectal, intravenous, subcutaneous, intraperitoneal, transdermal, etc. However oral administration is preferred.

The compositions for use according to the invention may be in conventional administration forms, eg. capsule, tablet, coated tablet, solution, suspension, syrup or suppository, and conventional formulation aids and excipients may be used, eg. viscosity modifiers, buffers, flavouring agents, suspension agents, stabilizers, excess ligand, and other additives.

The compositions of the invention may also contain other therapeutically active agents, eg. antidiabetic agents. In this regard, special mention may be made of the metal compounds considered to be useful in the treatment of diabetes, e.g. MgCl₂. Vanadium may of course be used in combination therapy with insulin, e.g. in the treatment of Type I diabetes.

Depending on the animal and condition being treated and on the administration route the vanadium compounds will generally be administered in dosages of 0.00035 to 600 mg V/kg bodyweight per day. The range is broad since in general the efficacy of a therapeutic effect for different mammals varies widely with doses typically being 20, 30 or even 40 times smaller (per unit bodyweight) in man than in the rat. Similarly the mode of administration can have a large effect on dosage. Thus for example oral dosages in the rat may be ten times the injection dose. As a result, the preferred range for rats is 0.1 to 300 mg V/kg/day while for man it may be 0.0007 to 2.0 mg V/kg/day.

The compositions, if in solution preferably contain the vanadium compounds at concentrations which provide the effective dose either in normal daily water intake or as a daily solution supplement(s) or tablet that provides the correct dose.

The vanadium compounds will generally be administered in dosages of 10⁻⁵ to 1000 mg V/kg/day bodyweight, especially 10⁻⁴ to 500 mg V/kg/day, particularly up to 400 mg V/kg/day and most especially up to 300 mg V/kg/day. Dosages for humans by oral administration will generally lie in the range 10⁻⁴ to 200 mg V/kg/day, e.g. 10⁻⁴ to 100 mg V/kg/day and by injection in the range 10⁻⁴ to 100 mg V/kg/day, e.g. 10⁻⁴ to 50 mg V/kg/day. For rats the minimum effective doses lie generally in the ranges 10 to 30 mg V/kg for oral administration and 6 to 20 mg V/kg for injection. Thus, for example, appropriate injection dosages for rats bis(maltolato)oxovanadium(IV) may be about 15 mg V/kg and appropriate oral dosages 150-300 mg/kg.

The compositions, if in solution form preferably contain the vanadium compounds at concentrations of 0.1 mM to 10 M, especially 0.2 mM to 1 M, particularly 1.6 to 3.2 mM.

Where the compositions are to be injected, the carrier for the vanadium compound may conveniently be aqueous methyl cellulose containing about 1% methyl cellulose.

The following examples are provided to illustrate the invention further.

### Example :

### Bis(maltolato)oxovanadium(IV) (BMOV)

The compound bis(maltolato)oxovanadium(IV) (hereinafter BMOV) was prepared nearly quantitatively by combining maltol (3-hydroxy-2-methyl-4-pyrone) and vanadyl sulfate in hot or boiling water at a ratio of 2 to 1. The pH of the solution was raised to 8.5 and the solution was refluxed overnight. The product was a deep purple-green compound that precipitated and was filtered after cooling the reaction mixture to ambient temperature. The compound is birefringent.

The compound is characterised as follows:

Its elemental analysis is correct for C₁₂H₁₀0₇V; % calculated (found): C 45.45 (45.60): H 3.18 (3.30) and its electron impact mess spectrum is also consistent: m/e = 317 (M⁺). The infrared spectrum shows absorptions (KBr disk) characteristic of the maltolato anion bound to a metal cation (1610, 1570, 1560, 1465 cm⁻¹) and characteristic of the vanadyl group V=O (995 cm⁻¹). It has a magnetic moment in the solid state of 1.76 B.M. indicating one unpaired electron; however, it gives a clear ¹H NMR spectrum in D₂O or d₄-methanol (δ 2.5 (s,6H), 6.55(d,2H), 8.15(d,2H)) and a clear ⁵¹V NMR spectrum in D₂O(-496 ppm). The compound is quite water-soluble (about 7 mM, 2mg mL⁻¹). Its log p value is 0.12.

The following experiments were conducted in rats with BMOV and vanadyl sulphate, the BMOV as prepared above.

The results are presented in part in the accompanying drawings in which:
Figure 1 relates change in weight with time;
Figure 2 shows average blood glucose values in mmol/l (over time);
Figure 3 shows the daily food consumption;
Figure 4 shows daily fluid consumption;

### Experiment 1

Initial experiments were carried out to determine pharmacological effectiveness of BMOV. Using male rats, made diabetic by the injection of streptozoticin (STZ) at a dose of 60 mg/kg i.v., BMOV was initially given by intraperitoneal (i.p.) injection as a suspension in 1% methyl cellulose.

### 1. Injection

Nine out of the twelve rats given 15 mg/kg i.p. (0.05 mmol/kg) responded to the compound with a decrease in blood glucose. Two animals developed hypoglycemia.

### 2. Oral Administration

a. Drinking. Administration of the vanadyl compound in the drinking water at doses of 0.46-0.92 mmol/kg (150-300 mg/kg, using concentrations of 0.5-1.3 mg/mL) reduced blood glucose in four diabetic rats into the normal range. Fluid intake was also decreased to normal in these animals.
b. Gavage. Six out of six diabetic rats responded to the vanadyl compound with a decrease in blood glucose when BMOV was given by gavage in a dose of 160 µmol/kg over 40 days. The peak fall in blood glucose occurred 5 hours after administering the drug. The blood glucose returned to diabetic levels usually within 12 hours.

Based on these preliminary studies, more detailed studies were undertaken to determine the minimum effective dose of BMOV required to maintain normal blood glucose in diabetic animals.

### Experiment 2

### Prolonged administration

BMOV was administered to normal and diabetic rats in drinking water.

The study was composed of 4 groups of animals. Control (8 animals), Control-Treated (11 animals), Diabetic (11 animals) and Diabetic-Treated (12 animals). The diabetic state was induced by injecting STZ at 60 mg/kg dissolved in 0.9% NaCl I.V. via the tail vein to anaesthetised rats. The two control groups were injected with 0.9% NaCl I.V. through the tail vein. The diabetic state was determined with a "Testape"™ test at 3 days post-injection and later confirmed with a glucometer test. Blood glucose and insulin assays were carried out post-injection over the course of the study. Treatment according to the invention was started 1 week after determination of the diabetic state.

Treated diabetic animals received between 0.3 and 0.5 mmol/kg of the compound/day in drinking water over a 77 day period. Treated control animals received a slightly lower dose (0.2-0.3 mmol/day) over the same period. The concentration of BMOV in the drinking water was varied between 1.6 and 3.2 mM. The reason for the differing doses in the two sets of animals is that the two groups drank different amounts of water daily; it was difficult to attain the same dose in both groups.

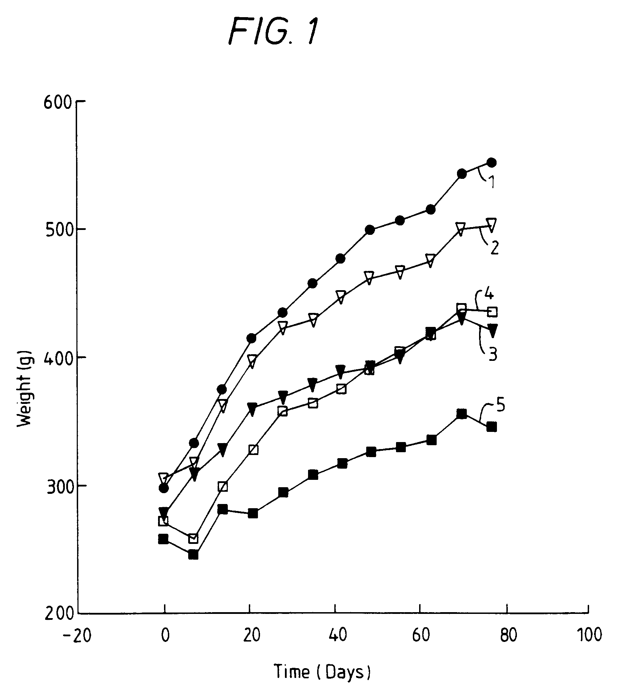

With the above treatment regimen, the following was noted:
1. The weight gain over the 77 day period is shown in Figure 1. As indicated above, 4 groups of animals were studied, but the diabetic-treated group was sub-divided to diabetic-treated responders (8 animals) and diabetic-treated non-responders (4 animals). Curve 1 is the control group, curve 2 the control-treated group, curve 3 the diabetic group, curve 4 is the diabetic-treated responders and curve 5 is the diabetic-treated non-responders. Initially, there was a significant difference only between the two control groups with respect to the three diabetic groups. However, by day 7 there was a significant difference between the two diabetic-treated groups with respect to all other groups. By day 28, the diabetic-treated non-responder group was significantly different from the other 4 groups and there was no longer a significant difference between the diabetic group and the diabetic responder group. By day 56, there was a significant difference between the control group and the control treated group. Treatment began with a 3.17 mM solution of the compound. On day 6, the concentration was reduced to 1.58 mM (0.5 mg/ml). On day 24, the concentration was increased to 2.37 mM (0.7 mg/ml). At this point 8 out of the 12 animals were responding to the compound.
The control treated animals showed a significantly decreased weight gain beginning at day 56. The decrease was correlated with the decrease in food intake.

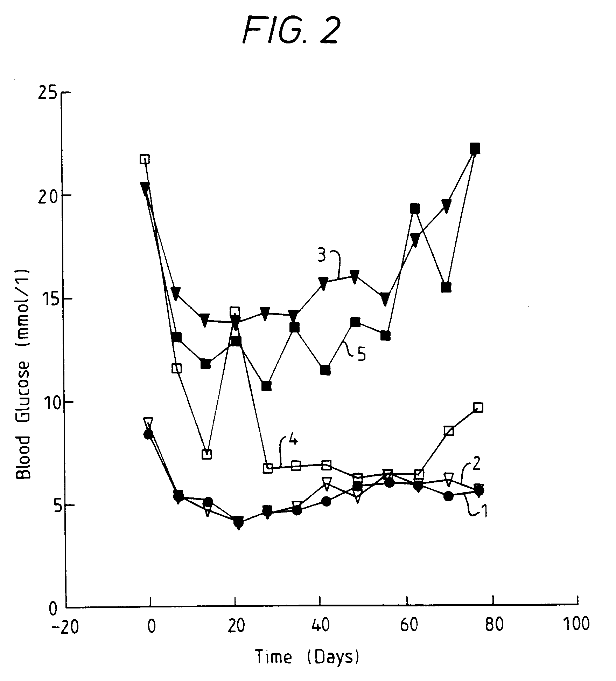

2. Figure 2 shows average blood glucose values (mmol/l) for the 5 groups based on weekly blood glucose determinations. The 5 groups were as in Figure 1. Initially, there was a significant difference between the two control groups with respect to all three of the diabetic groups. By day 7, there was a significant difference between the diabetic group with respect to both diabetic-treated groups. By day 14, the diabetic-treated responder group was euglycemic; however, on day 18 there was an increase in blood glucose levels for this group due to the necessity to withhold treatment for a few days to treat hypoglycemia, which developed in several animals. The diabetic-treated non-responder group consists of rats which exhibited marked fluctuations in glucose values.
Thus, eight of the twelve treated diabetic rats had their blood glucose values lowered from 20+mM to less than 10 mM from day 7 onwards. Four of the twelve rats had blood glucose readings ranging from 6-20mM on any given day. These rats are the non-responders. By day 24, diabetic rats that responded to the compound had normal blood glucose.

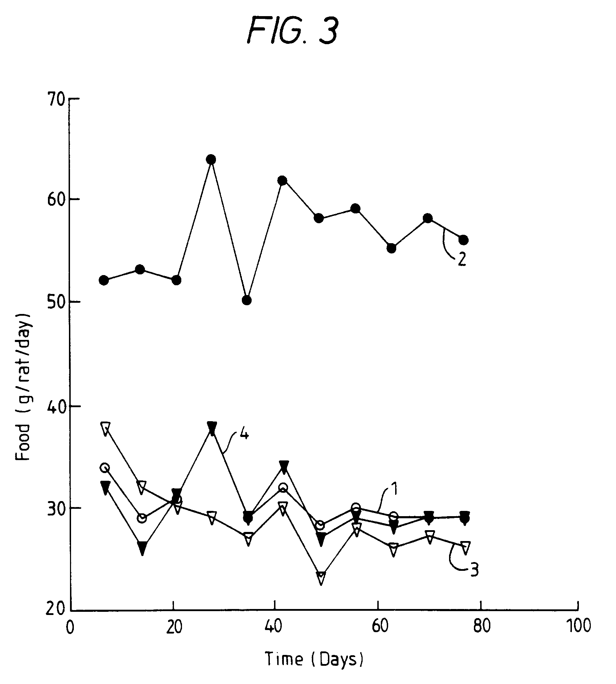

3. Figure 3 demonstrates the daily food consumption per rat. The rats were allowed free access to food with 2-3 rats per cage for the four treatment groups. The control group is curve 1, the diabetic group is curve 2, the control-treated group is curve 3 and the diabetic-treated group is curve 4. For the first approximately 50 days of treatment, the only significant difference occurred between the diabetic group with respect to all of the other groups. However, from day 63 on, there was also a significant difference between the control-treated group and all the other groups. There was no significant difference between the control and the diabetic-treated groups at any time.

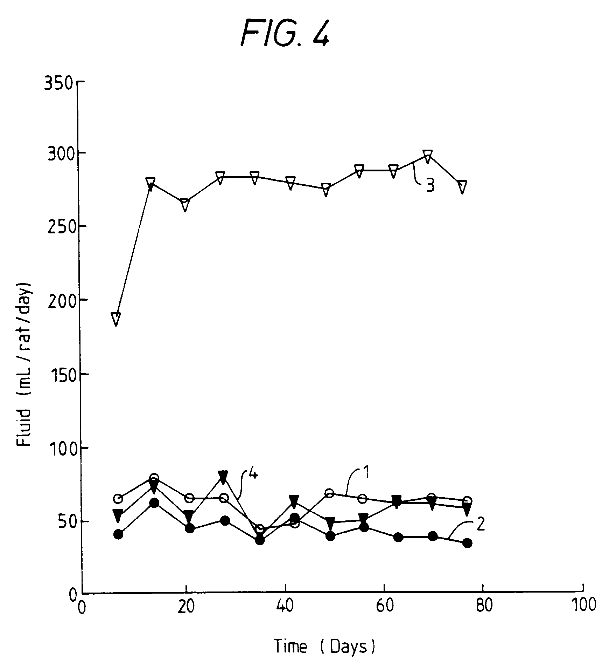

Figure 4 shows the daily fluid consumption per rat. The rats had free access to fluids with 2-3 rats per cage for the four treatment groups. The control group is curve 1, the control-treated group is curve 2, the diabetic group is curve 3 and the diabetic-treated group is curve 4. Initially, the only significant difference occurred between the diabetic group with respect to all of the other treatment groups. However by day 63, there was also a significant difference between the control treated group and all the other treatment groups. There was no significant difference in fluid consumption between the control and the diabetic-treated groups throughout.
Figures 3 and 4 illustrate that control of blood glucose was accompanied by a reduction in food intake in the diabetic rats from greater than 50 grams per day to about 30 grams per day (28±1.6 on day 77). There was a slight reduction in food intake (from 29± 0.1 to 25.5± 0.3) in control rats treated with the compound. Fluid intake fell from about 275 ml/rat in the diabetic group to about 60 ml/rat in the diabetic treated group. There was also a decrease in fluid intake in the control treated group compared with control rats (62.7±7.4 vs 33.6±7.2 ml on day 77). As stated above, the decrease in food and water intake correlates with the decrease in weight gain in control animals.
4. Treatment with BMOV decreased weight gain in control animals (200g vs 250g for control over the 77 day period). Diabetic-treated animals gained almost exactly the same weight (140g) as diabetics, despite the decrease in food intake; thus weight gain in diabetic-treated animals lagged behind that of control groups animals.
5. Insulin values in control treated rats decreased to the same value as those for diabetic animals (approximately 22 µU/ml) and were significantly lower than those of control (35.8±1.2 µU/ml) as shown in Table I:

**Table I**

| Insulin Values, µU/ml (Day 28 of BMOV study) | | | |
|---|---|---|---|
| Control | Control-Treated | Diabetic | Diabetic-Treated |
| 35.8±1.2 | 21.6±1.2 | 21.4±2.6 | 22.0±1.6 |

6. Plasma triglyceride and cholesterol values in diabetic animals were restored to control values by treatment with BMOV as shown in Table II:

**Table II**

| Lipid Values in BMOV Study Cholesterol mmol/l Means ± S.E.M. | | | | | |
|---|---|---|---|---|---|
| | Control | Control Treated | Diabetic-Treated Responders | Diabetic-Treated Non-Responders | Diabetic |
| Pretreatment (Day before treatment started) | 1.40±0.03 (8) | 1.64±0.08 (8) | 1.67±0.10 (7) | 1.51±0.07 (4) | 1.58±0.10 (11) |
| Week 6 | 1.37±0.06 (8) | 1.60±0.06 (10) | 1.58±0.11 (8) | 1.48±0.09 (4) | 2.65±0.28* (11) |

| Triglyceride mmol/l Means ± S.E.M. | | | | | |
|---|---|---|---|---|---|
| | Control | Control Treated | Diabetic-Treated Responders | Diabetic-Treated Non-Responders | Diabetic |
| Pretreatment (Day before treatment started) | 1.27±0.08 (8) | 1.21±0.14 (8) | 1.10±0.09 (8) | 1.85±0.41* (4) | 1.21±0.08 (11) |
| Week 6 | 1.81±0.11 (8) | 2.04±0.22 (10) | 1.70±0.18 (8) | 2.31±0.19 (4) | 4.14±0.98* (11) |

| | | | | | |
|---|---|---|---|---|---|
| * Diabetic untreated is significantly different from all other groups by ANOVA followed by either Fishers', Newman-Keuls' or Duncans' test. | | | | | |
| * Diabetic-treated nonresponders are significantly different from all other groups by ANOVA followed by either Fishers', Newman-Keuls' or Duncans' test. | | | | | |
| * Diabetic-untreated are significantly different from all groups except diabetic-treated nonresponders by ANOVA followed by Fishers' and Duncans' test, but not by ANOVA followed by Newman-Keuls' test. One rat in this group had a very high triglyceride value - 13.1 mmol/l. | | | | | |

7. At the end of 77 days of treatment, 0/8 of the treated diabetic rats, which were controlled by the drug, had cataracts. 5/11 Of the untreated diabetic animals showed cataracts. One of the four treated animals, not controlled by the drug, also had a cataract. The first cataract developed at 60 days in the untreated diabetic group.

The present invention provides a pharmaceutical composition useful for the treatment of diabetes (especially type II diabetes). The active compounds are absorbed across the gastrointestinal barrier and deliver the vanadyl ion to the bloodstream, where the insulin-mimetic properties of vanadium can be expressed. In contrast to insulin, the compositions are active when taken by mouth, and represent a significant advance in diabetes therapy. The active ingredients of the present invention are simple, monomeric species in the solid state. They are easily prepared, easily administered, relatively stable, and highly effective in lowering blood glucose.

## Claims

1. A pharmaceutical composition comprising at least one physiologically acceptable carrier together with a physiologically tolerable vanadium complex of a bidentate monoprotic α-hydroxypyrone or α-hydroxypyridinone chelant capable of chelating vanadium to form a five-membered, unsaturated, vanadium-containing ring, said ring containing at least two further heteroatoms.

2. A composition as claimed in claim 1 wherein said chelant is maltol.

3. A composition as claimed in either of claims 1 and 2 wherein said complex is vanadium (IV) or (V) complex.

4. A composition as claimed in any one of claims 1 to 3 wherein said complex is an oxovanadium (IV) complex.

5. A composition as claimed in any one of claims 1 to 4 in a form adapted for parenteral administration by injection.

6. A composition as claimed in any one of claims 1 to 4 in a form adapted for oral administration.

7. A composition as claimed in any one of claims 1 to 4 in a form adapted for subcutaneous, intraperitoneal or transdermal administration.

8. Bis(maltolato)oxovanadium(IV) for use in therapy.

9. The use of a vanadium complex as defined in any one of claims 1 to 4 for the manufacture of a therapeutic agent for lowering blood sugar levels.

## Patentansprüche

1. Pharmazeutische Zusammensetzung, die wenigstens einen physiologisch annehmbaren Träger umfaßt zusammen mit einem physiologisch tolerierbaren Vanadiumkomplex eines zweizähligen, einbasigen α-Hydroxypyron- oder α-Hydroxypyridinon-Chelatbildners, der zur Chelatisierung von Vanadium fähig ist, um einen fünfgliedrigen, ungesättigten, Vanadium enthaltenden Ring zu bilden, wobei der Ring wenigstens zwei weitere Heteroatome enthält.

2. Zusammensetzung nach Anspruch 1, worin der Chelatbildner Maltol ist.

3. Zusammensetzung nach Anspruch 1 oder 2, worin der Komplex ein Vanadium(IV)- oder -(V)-Komplex ist.

4. Zusammensetzung nach einem der Ansprüche 1 bis 3, worin der Komplex ein Oxovanadium(IV)-Komplex ist.

5. Zusammensetzung nach einem der Ansprüche 1 bis 4 in einer Form, die der parenteralen Verabreichung durch Injektion angepaßt ist.

6. Zusammensetzung nach einem der Ansprüche 1 bis 4 in einer Form, die der oralen Verabreichung angepaßt ist.

7. Zusammensetzung nach einem der Ansprüche 1 bis 4 in einer Form, die der subkutanen, intraperitonealen oder transdermalen Verabreichung angepaßt ist.

8. Bis(maltolato)oxovanadium(IV) zur Verwendung in der Therapie.

9. Verwendung eines Vanadiumkomplexes, wie in einem der Ansprüche 1 bis 4 definiert, zur Herstellung eines therapeutischen Mittels zur Verringerung von Blutzuckerspiegeln.

## Revendications

1. Composition pharmaceutique comprenant au moins un véhicule physiologiquement acceptable associé à un complexe de vanadium physiologiquement tolérable d'un agent chelatant monoprotique bidente de type α-hydroxypyrone ou α-hydroxypyridinone capable de chelater le vanadium pour former un cycle insaturé à cinq membres contenant du vanadium, ledit cycle contenant au moins deux hetéroatomes supplémentaires.

2. Composition selon la revendication 1, dans laquelle ledit agent chelatant est le maltol.

3. Composition selon la revendication 1 ou 2, dans laquelle ledit complexe est un complexe de vanadium (IV) ou (V).

4. Composition selon l'une quelconque des revendications 1 à 3, dans laquelle ledit complexe est un complexe d'oxovanadium (IV)

5. Composition selon l'une quelconque des revendications 1 à 4, dans une forme appropriée pour l'administration parentérale par injection.

6. Composition selon l'une quelconque des revendications 1 à 4, dans une forme appropriée à l'administration orale.

7. Composition selon l'une quelconque des revendications 1 à 4, dans une forme appropriée pour l'administration sous-cutanée intrapéritonéale ou transdermique.

8. Bis (maltolato)oxovanadium (IV) pour une utilisation en thérapie.

9. Utilisation d'un complexe de vanadium selon l'une quelconque des revendications 1 à 4 , pour la fabrication d'un agent thérapeutique destiné à diminuer les taux de sucre dans le sang.
